# EUROPEAN PATENT SPECIFICATION

(11) **EP 0 862 570 B1**
(45) Date of publication and mention of the grant of the patent: **21.03.2001**
(21) Application number: 96938145.8
(22) Date of filing: 06.11.1996
(51) Int. Cl.: C07D 473/00

(54) **PROCESS FOR THE PREPARATION OF 9- (2-HYDROXYETHOXY)METHYL]GUANINE**
VERFAHREN ZUR HERSTELLUNG VON 9-(2-HYDROXYETHOXY)METHYL]GUANINE
PROCEDE DE PREPARATION DE 9- (2-HYDROXYETHOXY)METHYL]GUANINE

(30) Priority: 14.11.1995 IT MI952333
(43) Date of publication of application: 09.09.1998
(73) Proprietor: Clariant Life Science Molecules (Italia) S.p.a., 20124 Milano (IT)
(72) Inventor: BELLANI, Piero, I-20017 Rho (IT)
(74) Representative: Dragotti, Gianfranco
(86) International application number: EP9604882
(87) International publication number: WO9718211

(56) References cited:
- EP-A- 0 289 992
- EP-A- 0 532 878
- EP-A- 0 564 006
- EP-A- 0 628 558
- GB-A- 1 567 671
- US-A- 4 146 715
- CHEMICAL & PHARMACEUTICAL BULLETIN, vol. 36, no. 3, March 1988, pages 1153-1157, XP002024969 HIROATSU MATSUMOTO ET AL: cited in the application

## Description

The present invention relates to a process for the preparation of 9-[(2-hydroxyethoxy)methyl] guanine, known under its International Non-proprietary Name "acyclovir", hereinbelow designated by this name and represented by the following structural formula I

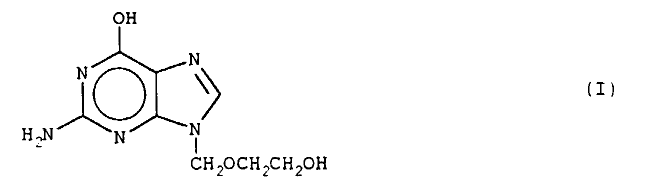

Acyclovir has been disclosed for the first time in Belgian patent 833,006; in this document there are also described various processes for its preparation, including the process involving the final step of transformation of the O-protected acyclovir into acyclovir. The expression "O-protected acyclovir" designates the protected acyclovir on the aliphatic hydroxy group. According to this method, the O-protecting group is, in general, an ester, more particularly an acyl group, preferably a benzoyl group. Removal of the O-protecting group is carried out, according to BE 833,006, by treatment with ammonia saturated methanol to give acyclovir in a 75% yield.

Other methods for the preparation of acyclovir are indicated by the literature. Thus, EP 532,878 discloses the preparation of o,N²-diprotected acyclovir by acetylation and subsequent deprotection by treatment with sodium hydroxyde to give acyclovir in a 92% yield. According to this document, however, diacetylacyclovir [N²-acetyl-9-(2-acetoxyethoxymethyl)guanine] is in its turn obtained in admixture with N²-acetyl-7-(2-acetoxyethoxymethyl)guanine in the 2.5:1 ratio which requires a previous chromatographic separation of the two isomers.

An analogous synthesis is described by H. Matsumoto et al. in Chem. Pharm. Bull. 1988, 36, 1153, where, after the separation of the two acetylated isomers, the hydrolysis of diacetylacyclovir is carried out with aqueous ammonia to give acyclovir in a 55% yield.

In EP 564,006, the preparation of 9-(2-acetoxyethoxymethyl)guanine and N²-acetyl-9-(2-acetoxyethoxymethyl)guanine is described. The conversion into acyclovir is carried out with a basis, particularly with ammonia, a 50% aqueous solution of methylamine, sodium methylate or sodium hydroxide even though it is not exemplified.

The document IT MI 93A001264 discloses a process for obtaining acyclovir which is substantially analogous to that described in BE 833,006, whereby O-protected acyclovir is used as starting material, the protecting group being acyl and the deprotection being carried out with sodium hydroxide.

The above mentioned documents and in general the known methods for the preparation of acyclovir involve, at the end of the synthesis, complex purifications, more particularly a passage of very diluted aqueous solutions of the product on columns of ionic resins, which render such methods somewhat laborious.

It has now been found that acyclovir in a pharmacologically acceptable purity state can be prepared in very high yields by hydrolysis of diacetylacyclovir of formyla II 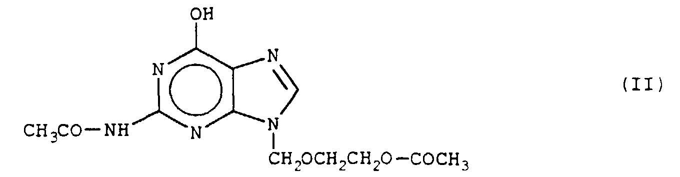 by hydrolysis with an aqueous solution of monoethanolamine.

It has also surprisingly found that, by hydrolysis with aqueous monoethanolamine, acyclovir precipitates straightforward in the reaction medium and may be thus isolated by simple filtration in a purity state at least sufficient to be pharmacologically acceptable.

Finally, it has been found that acyclovir may be easily purified by transformation in one of its alkali metal salts of formula III 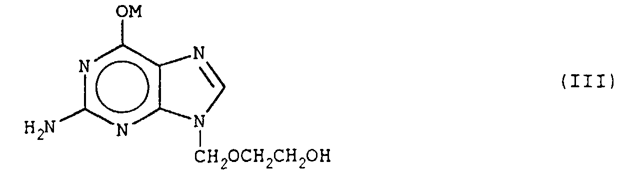 wherein M is an atom of an alkali metal, preferably sodium or potassium, and subsequent neutralization of the aqueous solution of said salt.

Thus, according to one of its aspects, the present invention concerns a process for the preparation of acyclovir which comprises
(a) treating the N²-acetyl-9-(2-acetoxyethoxymethyl)guanine (diacetylacyclovir) with monoethanolamine in aqueous solution at a temperature of 70-95°C;
(b) isolating the acyclovir thus obtained by treatment with a mineral or organic acid and subsequently filtering the separated precipitate.

Step (a) is carried out starting from a N²-acetyl-9-(2-acetoxy ethoxymethyl)guanine which may be pure or in a wet state by the residual solvent of its synthesis, in aqueous medium at a temperature of 70-95°C. At this temperature the reaction is complete after about 3 hours.

In step (b), after the possible removal of the residual solvent, for example by distillation under vacuum, the reaction mixture is treated with an acid, for example with acetic acid, up to neutrality. Acyclovir thus obtained is isolated by filtration and subsequent washing with an appropriate solvent, for example a ketone such as acetone, methylethylketone or methylisobutylketone, and final drying.

In general, the product obtained is of great purity, read for use in pharmaceutical formulations.

If a further increase of the purity degree or if an acyclovir obtained by another route is available, the product may be, in general, purified by transformation in one of its alkali metal salts, preferably in its sodium salt and subsequent neutralization in aqueous medium. Such a purification presents the advantage that it can be carried out on any crude acyclovir obtained according to any other method.

According to another of its aspects, the present invention provides a method for the purification of acyclovir which comprises
(c) treating acyclovir with an alkaline basis; and
(d) neutralizing the thus isolated salt by addition of an acid in aqueous medium and isolating the pure acyclovir thus obtained by filtration.

In step (c) the crude acyclovir, for example the product obtained at the end of the above illustrated step (b), even if it is wet by washing solvent, is treated with an alkaline basis, for example with sodium or potassium hydroxide or with sodium or potassium carbonate, in aqueous medium and an organic solvent, preferably a ketone such as acetone, methylethylketone or methylisobutylketone is added to the clear solution; preferably this solvent is the same which has been used for washing acyclovir, when this product has been prepared according to the above illustrated steps (a) and (b).

The acyclovir alkali metal salt thus obtained is isolated by precipitation according to conventional methods, by decolorizing with carbon, if needed, the solution of the salt before the precipitation, then filtering the product and washing it. It is employed as such in the subsequent step.

In step (d) the alkali metal salt thus obtained is neutralized in aqueous medium with a mineral or organic acid, for example with hydrochloric or hydrobromic, acetic, propionic or methanesulfonic acid and the acyclovir in a high purity degree thus obtained is isolated by filtration, subsequent washing and drying.

According to a preferred aspect, the present invention finally concerns a process for the preparation of highly pure (≥ 99%) 9-(2-hydroxyethoxymethyl)guanine which comprises
(a) treating N²-acetyl-9-(2-acetoxyethoxymethyl)guanine of formula II with monoethanolamine in aqueous solution at a temperature of 70-95°C;
(b) isolating the 9-(2-hydroxyethoxymethyl)guanine thus obtained by neutralization with acetic acid and filtration of the separated precipitate;
(c) treating the wet 9-(2-hydroxyethoxymethyl)guanine with sodium hydroxide in aqueous medium and letting the sodium salt to precipitate, which is isolated by filtration;
(d) neutralizing the wet sodium salt thus obtained with acetic acid in aqueous medium and isolating the thus obtained very pure 9-(2-hydroxyethoxymethyl)guanine by filtration.

The process of the present invention, on one side, allows the preparation of acyclovir by a method which gives it straightforward in a pure crystalline state at the end of the deprotection reaction of the diacetylacyclovir and, on the other side, allows a further purification of acyclovir without using particular apparatus, expecially ionic resins columns.

The following examples illustrate the invention without, however, limiting it.

### EXAMPLE 1

A mixture of 46.250 g (0.149 moles) of N²-acetyl-9-(2-acetoxyethoxy methyl)guanine and 32.750 g (0.536 moles) of monoethanolamine in 600 ml of deionized water are heated at 90°C for 3 hours under stirring, then it is cooled to 60°C and 25 ml of 80% acetic acid are added thereinto to reach a pH of 6.8÷7.2. The mixture is further cooled to about 20°C, then 60 ml of water and 60 ml of acetone are added thereinto. The crystalline product is filtered and washed with acetone. Thus, there is obtained 50 g of acyclovir, wet of acetone, corresponding to 33.250 g of an acyclovir having purity characteristics corresponding to those of a USP standard. Yield: 98.7% of the theoretical.

### EXAMPLE 2

(a) To a mixture of 30 g of acyclovir, wet of acetone, obtained according to Example 1 and corresponding to 19.950 g of dry product, 36 ml of deionized water and 9 ml of 30% sodium hydroxide are added in order to have a pH of 11.8 - 12.0, then 37.5 ml of acetone and 150 ml of decolorizing charcoal are added into the solution. The mixture is filtered, by washing with 1.5 ml of deionized water and 1.5 ml of acetone, the solution is heated to 35°C and 195 ml of acetone are added thereinto. The suspension thus obtained is cooled to 15°C, stirred 2 hours at the same temperature, filtered and the product is washed with 35 ml of acetone. There is obtained 25.5 g of sodium salt, wet of acetone, corresponding to 23.2 g of dry product.
(b) To a solution of 24 g of acyclovir sodium salt, wet of acetone, as obtained according to (a) and corresponding to 21.9 g of dry product, in 220 ml of deionized water, 6.75 ml of 80% acetic acid are added. The suspension of the crystalline product thus obtained is cooled at 15°C and stirred for 1 hour. Then it is filtered, washed with 35 ml of deionized water and 35 ml of acetone, then dried under vacuum at 80°C up to a residual moisture not higher than 5%. So, there is obtained 18.2 g of acyclovir having a purity of 99.85%.

### EXAMPLE 3

By operating as described in Example 1, from 17.5 g of N²-acetyl-9-(2-acetoxyethoxymethyl)guanine 18.9 g of acyclovir, wet of acetone, are obtained. By drying under vacuum at 80°C up to constant weight, 12.650 g of acyclovir with a purity of 99.25% are obtained.

### EXAMPLE 4

By operating as described in Example 2, starting from 4.750 g of acyclovir with a purity of 95.47%, prepared according to Example 6 of BE 833,006, 5.08 g of sodium salt are obtained. By neutralization in aqueous solution of the sodium salt thus obtained, using 80% acetic acid, 4.220 g of acyclovir with a purity of 99.27% are obtained.

## Claims

1. A process for the preparation of 9-(2-hydroxyethoxymethyl)guanine which comprises:
(a) treating the N²-acetyl-9-(2-acetoxyethoxymethyl)guanine with monoethanolamine in aqueous solution at a temperature of 70÷95° C;
(b) isolating the 9-(2-hydroxyethoxymethyl)guanine thus obtained by treatment with a mineral or organic acid and filtering the separated precipitate.

2. A process according to claim 1 in which acetic acid is employed in step (b).

3. A process for the purification of 9-(2-hydroxyethoxymethyl)guanine obtained according to claim 1 which further comprises:
(c) treating the 9-(2-hydroxyethoxymethyl)guanine with an alkaline basis; and
(d) neutralizing the thus isolated alkaline salt by addition of an acid in aqueous medium and isolating the pure 9-(2-hydroxyethoxymethyl)guanine thus obtained.

4. A process according to claim 3 in which sodium or potassium hydroxyde is used as an alkaline basis in step (c).

5. A process according to claim 3 in which acetic acid is used in step (d).

## Patentansprüche

1. Ein Verfahren zur Herstellung von 9-(2-Hydroxyethoxymethyl) guanidin, welches
a) die Behandlung des N²-Acetyl-9-(2-acetoxyethoxymethyl)guanidins mit Monoethanolamin in wässriger Lösung bei einer Temperatur von 70-95°C;
(b) die Isolierung des so erhaltenen 9-(2-Hydroxyethoxymethyl)guanidins durch Behandlung mit einer anorganischen oder organischen Säure und Abfiltrieren des abgetrennten Niederschlages umfasst.

2. Ein Verfahren gemäss Patentanspruch 1, worin in der Stufe (b) Essigsäure verwendet wird.

3. Ein Verfahren zur Reinigung des gemäss Patentanspruch 1 erhaltenen 9-(2-Hydroxyethoxymethyl)guanidins, welches ferner
(c) die Behandlung des 9-(2-Hydroxyethoxymethyl)guanidins mit einer alkalischen Base; und
(d) die Neutralisierung des so isolierten alkalischen Salzes durch Zugabe einer Säure in wässrigem Medium und die Isolierung des so erhaltenen, reinen 9-(2-Hydroxyethoxymethyl)guanidins umfasst.

4. Ein Verfahren gemäss Patentanspruch 3, worin in der Stufe (c) als alkalische Base Natrium- oder Kaliumhydroxyd verwendet werden.

5. Ein Verfahren gemäss Patentanspruch 3, worin in der Stufe (d) Essigsäure verwendet wird.

## Revendications

1. Un procédé de préparation de la 9-(2-hydroxyéthoxyméthyl)-guanine, qui comprend
(a) le traitement de la N²-acétyl-9-(2-acétoxyéthoxyméthyl)-guanine par la monoéthanolamine en solution aqueuse à une température de 70-95°C;
(b) l'isolement de la 9-(2-hydroxyéthoxyméthyl)-guanine ainsi obtenue par traitement avec un acide minéral ou organique et filtration du précipité qui s'est séparé.

2. Un procédé selon la revendication 1, dans lequel on utilise l'acide acétique à l'étape (b).

3. Un procédé de purification de la 9-(2-hydroxyéthoxyméthyl)-guanine obtenue selon la revendication 1, qui comprend en outre:
(c) le traitement de la 9-(2-hydroxyéthoxyméthyl)-guanine par une base alcaline; et
(d) la neutralisation par addition d'un acide en milieu aqueux du sel alcalin ainsi isolé et l'isolement de la 9-(2-hydroxyéthoxyméthyl)-guanine pure ainsi obtenue.

4. Un procédé selon la revendication 3, dans lequel on utilise l'hydroxyde de sodium ou de potassium comme base alcaline à l'étape (c).

5. Un procédé selon la revendication 3, dans lequel on utilise l'acide acétique à l'étape (d).
